# EUROPÄISCHE PATENTSCHRIFT

(11) **EP 0 442 141 B1**
(45) Veröffentlichungstag und Bekanntmachung des Hinweises auf die Patenterteilung: **02.03.1994**
(21) Anmeldenummer: 90125410.2
(22) Anmeldetag: 24.12.1990
(51) Int. Cl.: A61K 31/35, A61K 45/06

(54) **Pharmazeutische Zubereitung**
Pharmaceutical preparation
Préparation pharmaceutique

(30) Priorität: 13.02.1990 DE 4004268
(43) Veröffentlichungstag der Anmeldung: 21.08.1991
(73) Patentinhaber: MERCK PATENT GmbH, D-64271 Darmstadt (DE)
(72) Erfinder: Gericke, Rolf, Dr., W-6104 Seeheim (DE); Baumgarth, Manfred, Dr., W-6100 Darmstadt (DE); Lues, Ingeborg, Dr., W-6100 Darmstadt (DE); Bergmann, Rolf, Dr., W-6101 Reichelsheim (DE)

(56) Entgegenhaltungen:
- EP-A- 0 323 745
- CHEMICAL ABSTRACTS, Band 107, Nr. 9, 31. August 1987, Seite 41, Zusammenfassung Nr. 70487r, Columbus, Ohio, US; R. KIRSTEN et al.: "Influence of different bisoprolol doses on hemodynamics, plasma catecholamines, platelet aggregation, and alpha2- and beta-receptors in hypertensive patients", & J. CARDIOVASC. PHARMACOL. 1986, 8(Suppl. 11). S113-S121

## Beschreibung

Gegenstand der Erfindung ist eine neue pharmazeutische Zubereitung enthaltend Bisoprolol und/oder eines seiner physiologisch unbedenklichen Salze und einen Kaliumkanalaktivator.

Diese neue Zubereitung hat blutdrucksenkende Wirkung und kann außerdem zur Behandlung von Asthma, peripheren Durchblutungsstörungen, Herzinsuffizienz und/oder Angina pectoris verwendet werden.

Der Erfindung lag die Aufgabe zugrunde, neue Arzneimittel in Form von pharmazeutischen Zubereitungen zur Verfügung zu stellen, die bessere Eigenschaften besitzen als bekannte für die gleichen Zwecke verwendbare Arzneimittel.

Diese Aufgabe wurde durch das Auffinden der neuen Zubereitung gelöst. Bisoprolol = (+)-1-p(2-Isopropoxyethoxymethyl)-phenoxy-3-isopropylamino-2-propanol ist aus der DE-OS 2 645 710 bekannt. Als Salze von Bisoprolol sind das Hydrochlorid und das Hemifumarat bevorzugt.

In der EP-A2-0323 745 sind bereits pharmazeutische Präparate beschrieben, die Kombinationen von Kaliumkanalaktivatoren mit β-Rezeptorenblockern enthalten. Unter den dort genannten β-Rezeptorenblockern ist Bisoprolol jedoch nicht aufgeführt. Im Vergleich zu den dort genannten Präparaten zeichnen sich die vorliegenden neuen Zubereitungen durch hohe β₁-Selektivität und lange Wirkungsdauer aus.

Kaliumkanalaktivatoren sind z.B. beschrieben in EP-A-0205 292, EP-A-0214 818, EP-A-0250 077, GB-A-1489 879, EP-A-0112 776, EP-A-0273-262, EP-A-0277 612, EP-A-0277 612, EP-A-0340 718 und EP-A-0346 724.

Bevorzugt sind Kaliumkanalaktivatoren der allgemeinen Formel 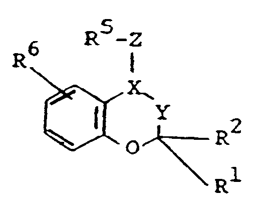
worin
- X: -CR⁴-,
- Y: -CHR³-,
- R¹ und R²: jeweils unabhängig voneinander A,
- R³: OH,
- R⁴: H,
- R³ und R⁴: zusammen auch eine Bindung,
- R⁵: einen unsubstituierten oder einen einfach durch A substituierten Oxodihydropyridyl- oder Oxo-dihydropyridazinyl-rest,
- R⁶: CN
- Z: eine Bindung oder O und
- A: Alkyl mit 1-6 C Atomen,

bedeuten,
und/oder eines seiner physiologisch unbedenklichen Salze.

Die Verbindungen der allgemeinen Formel sind teilweise bekannt (vgl. z.B. EP-A-0273 262).

Unter der Verbindungen der allgemeinen Formel sind diejenigen bevorzugt, in denen die Reste R¹ und R² jeweils CH₃ und/oder der Rest R⁶ CN bedeuten, wobei der Rest R⁶ vorzugsweise in 6-Stellung steht. Die Gruppierung X-Y bedeutet vorzugsweise -CH-CHOH-.

Die Gruppe R⁵-Z bedeutet vorzugsweise 1H-2-Pyridon-1-yl, 2-Hydroxy-4-pyridyl-oxy, 6-Hydroxy-3-pyridazinyl-oxy, 1,6-Dihydro-1-methyl- oder 1,6-Dihydro-1-ethyl-6-oxo-3-pyridazinyl-oxy.

Einzelne bevorzugte Verbindungen der allgemeinen Formel sind 2,2-Dimethyl-4-(1H-2-pyridon-1-yl)-6-cyan-3-chromanol (Ia), insbesondere dessen (3S,4R)-Enantiomeres, 2,2-Dimethyl-4-(1H-2-pyridon-1-yl)-6-cyan-2H-chromen (Ib), 2,2-Dimethyl-4-(6-hydroxy-3-pyridazinyl-oxy)-6-cyan-3-chromanol, insbesondere dessen (3S,4R)-Enantiomeres 2,2-Dimethyl-4- (1, 6-dihydro-1-methyl-6-oxo-3-pyridazinyl-oxy)-6-cyan-3-chromanol (Ic), insbesondere dessen (3S,4R)-Enantiomeres.

Gleichzeitige Gabe von Bisoprolol verstärkt bemerkenswerterweise deutlich die bluttdrucksenkende Wirkung der genannten Kaliumkanalaktivatoren. Die Wirkung der Kombination ist größer als nach Abschätzung der Wirkung der Einzelkomponenten erwartet werden kann. Dieser (synergistische) Effekt kann z.B. in Standardtests an narkotisierten oder wachen Ratten, Hunden, Katzen, Affen oder Minischweinen ermittelt werden, z.B. nach Methoden, wie sie in der EP-A2-0323 745 beschrieben sind.

Die neue pharmazeutische Zubereitung kann hergestellt werden, indem man Bisoprolol und/oder eines seiner physiologisch unbedenklichen Salze und (mindestens) einen Kaliumkanalaktivator, der allgemeinen Formel, zusammen mit mindestens einem festen, flüssigen oder halbflüssigen Träger- oder Hilfsstoff in eine geeignete Dosierungsform bringt. Die so erhaltene Zubereitung kann als Arzneimittel in der Human- oder Veterinärmedizin, insbesondere bei der Senkung des Blutdrucks, eingesetzt werden. Als Trägersubstanzen kommen organische oder anorganische Stoffe in Frage, die sich für die enterale (z.B. orale, sublinguale oder rektale), parenterale oder topische (z.B. transdermale) Applikation eignen und mit den Wirkstoffen nicht reagieren, beispielsweise Wasser, pflanzliche Öle, Benzylalkohole, Polyethylenglykole, Glycerintriacetat und andere Fettsäureglyceride, Gelatine, Sojalecithin, Kohlehydate wie Lactose oder Stärke, Magnesiumstearat, Talk, Cellulose. Zur oralen Anwendung dienen insbesondere Tabletten, Dragees, Kapseln, Sirupe, Säfte oder Tropfen, zur rektalen Anwendung Suppositorien, zur parenteralen Applikation Lösungen, vorzugsweise ölige oder wässerige Lösungen, ferner Suspensionen, Emulsionen oder Implantate, für die topische Anwendung Salben, Cremes oder Pflaster. Die Wirkstoffe können auch lyophilisiert und die so erhaltenen Lyophilisate z.B. zur Herstellung von Injektionspräpararten verwendet werden. Die Zubereitungen können sterilisiert sein und/oder Hilfsstoffe wie Konservierungs-, Stabilisierungs- und/oder Netzmittel, Emulgatoren, Salze zur Beeinflussung des osmotischen Druckes, Puffersubstanzen, Farb- und/oder Aromastoffe enthalten. Sie können auch weitere Wirkstoffe enthalten, z.B. andere blutdrucksenkende oder diuretisch wirkende Stoffe.

Die erfindungsgemäßen Zubereitungen werden bei der Senkung des Blutdrucks und/oder der Bekämpfung von Krankheiten verwendet. In der Regel werden sie zur Therapie und/oder Prophylaxe von Asthma, Störungen des cardiovasculären Systems, insbesondere dekompensierter Herzinsuffizienz, Angina pectoris, peripheren oder cerebralen Gefäßerkrankungen sowie krankheitszuständen, die mit Bluthochdruck verbunden sind, in Analogie zu bekannten blutdrucksenkend wirksamen Substanzen, insbesondere β-Rezeptorenblockern oder den Kaliumkanalaktivatoren selbst, verabreicht. Die Dosierungen der Verbindungen der allgemeinen Formel bzw. ihrer Salze liegen vorzugsweise zwischen etwa 0,1 mg und 50 mg, insbesondere 0,2 und 10 mg, ganz besonders bevorzugt zwischen 0,1 und 5 mg pro Dosierungseinheit. Bisoprolol wird vorzugsweise in Dosierungen zwischen etwa 1 und etwa 100 mg, insbesondere zwischen 2 und 20 mg pro Dosierungseinheit verwendet. Die tägliche Dosierung der Verbindungen der allgemeinen Formel bzw. ihrer Salze liegt vorzugsweise zwischen etwa 0,001 und 1, insbesondere zwischen 0,002 und 0,2 mg/kg Körpergewicht, diejenige des Bisoprolols vorzugsweise zwischen etwa 0,02 und 0,2 mg/kg Körpergewicht. Die spezielle Dosis für jeden bestimmten Patienten hängt jedoch von den verschiedensten Faktoren ab, beispielsweise von der Wirksamkeit der eingesetzten speziellen Verbindung, vom Alter, Körpergewicht, allgemeinen Gesundheitszustand, Geschlecht, von der Kost, vom Verabfolgungszeitpunkt und -weg, von der Ausscheidungsgeschwindigkeit, Arzneistoffkombination und Schwere der jeweiligen Erkrankung, welcher die Therapie gilt. Die orale Applikation ist bevorzugt.

Die Bestandteile der neuen pharmazeutischen Zubereitung werden vorzugsweise kombiniert verabreicht. Sie können aber auch einzeln gleichzeitig oder aufeinanderfolgend verabreicht werden.

### Beispiel 1: Tabletten

Ein Gemisch von 20 g Ia, 100 g Bisoprolol-hemifumarat, 4 kg Lactose, 1,2 kg Kartoffelstärke, 0,2 kg Talk und 0,1 kg Magnesiumstearat wird in üblicher Weise zu Tabletten gepreßt, derart, daß jede Tablette 1 mg Ia und 5 mg Bisoprolol-hemifumarat enthält.

### Beispiel 2: Dragees

Analog Beispiel 1 werden Tabletten gepreßt, die anschließend in üblicher Weise mit einem Überzug aus Saccharose, Kartoffelstärke, Talk, Tragant und Farbstoff überzogen werden.

### Beispiel 3: Kapseln

Man füllt ein gesiebtes Gemisch von 30 g Ib, 100 g Bisoprolol-hemifumarat und 6 kg Lactose, in üblicher Weise in Hartgelatinekapseln, so daß jede Kapsel 0,3 mg Ib und 1 mg Bisoprolol-hemifumarat enthält.

### Beispiel D: Ampullen

Eine Lösung von 2 g Ic und 20 g Bisoprolol-hemifumarat in 30 l zweifach destilliertem Wasser wird steril filtriert, in Ampullen abgefüllt, unter sterilen Bedingungen lyophilisiert und steril verschlossen. Jede Ampulle enthält 0,1 mg Ic und 1 mg Bisopolol-hemifumarat.

## Patentansprüche (Patentansprüche für folgende(n) Vertragsstaat(en): AT, BE, CH, DE, DK, FR, GB, GR, IT, LI, LU, NL, SE)

1. Pharmazeutische Zubereitung enthaltend Bisoprolol und/oder eines seiner physiologisch unbedenklichen Salze und einen Kaliumkanalaktivator der allgemeinen Formel 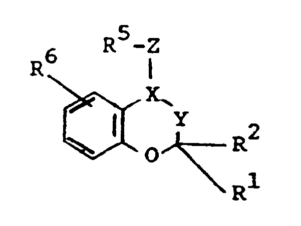 worin
X -CR⁴-,
Y -CHR³-,
R¹ und R² jeweils unabhängig voneinander A,
R³ OH,
R⁴ H
R³ und R⁴ zusammen auch eine Bindung,
R⁵ einen unsubstituierten oder einen einfach durch A substituierten Oxodihydropyridyl- oder Oxo-dihydropyridazinyl-rest,
R⁶ CN
Z eine Bindung oder O und
A Alkyl mit 1-6 C Atomen,
bedeuten,
und/oder eines seiner physiologisch unbedenklichen Salze.

2. Verfahren zur Herstellung einer pharmazeutischen Zubereitung, dadurch gekennzeichnet, daß man Bisoprolol und/oder eines seiner physiologisch unbedenklichen Salze und einen Kaliumkanalaktivator der allgemeinen Formel zusammen mit mindestens einem festen, flüssigen oder halbflüssigen Träger- oder Hilfsstoff in eine geeignete Dosierungsform bringt.

3. Verwendung einer pharmazeutische Zubereitung, enthaltend Bisoprolol und/oder eines seiner physiologisch unbedenklichen Salze und einen Kaliumkanalaktivator der allgemeinen Formel zur Herstellung eines Arzneimittels zur Senkung des Blutdruckes.

## Patentansprüche (Patentansprüche für folgende(n) Vertragsstaat(en): ES)

1. Verfahren zur Herstellung einer pharmazeutischen Zubereitung, enthaltend Bisoprolol und/oder eines seiner physiologisch unbedenklichen Salze und einen Kaliumkanalaktivator der allgemeinen Formel 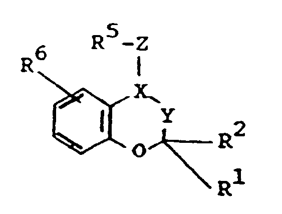 worin
X -CR⁴-,
Y -CHR³-,
R¹ und R² jeweils unabhängig voneinander A,
R³ OH,
R⁴ H,
R³ und R⁴ zusammen auch eine Bindung,
R⁵ einen unsubstituierten oder einen einfach durch A substituierten Oxodihydropyridyl- oder Oxo-dihydropyridazinyl-rest,
R⁶ CN
Z eine Bindung oder O und
A Alkyl mit 1-6 C Atomen,
bedeuten,
und/oder eines seiner physiologisch unbedenklichen Salze, dadurch gekennzeichnet, daß man Bisoprolol und/oder eines seiner physiologisch unbedenklichen Salze und einen Kaliumkanalaktivator der allgemeinen Formel zusammen mit mindestens einem festen, flüssigen oder halbflüssigen Träger- oder Hilfsstoff in eine geeignete Dosierungsform bringt.

## Claims (Claims for the following Contracting State(s): AT, BE, CH, DE, DK, FR, GB, GR, IT, LI, LU, NL, SE)

1. Pharmaceutical composition containing bisoprolol and/or one of its physiologically acceptable salts and a potassium channel activator of the general formula 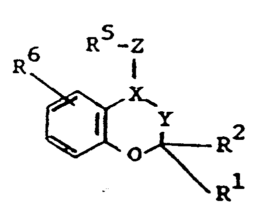 in which
X is -CR⁴-,
Y is -CHR³-,
R¹ and R² are each independently of one another A,
R³ is OH,
R⁴ is H,
R³ and R⁴ together are also a bond,
R⁵ is an oxodihydropyridyl or oxodihydropyridazinyl radical which is unsubstituted or substituted once by A,
R⁶ is CN,
Z is a bond or 0, and
A is alkyl with 1-6 C atoms
and/or one of the physiologically acceptable salts thereof.

2. Process for the preparation of a pharmaceutical composition, characterized in that bisoprolol and/or one of its physiologically acceptable salts and a potassium channel activator of the general formula are converted together with at least one solid, liquid or semi-liquid vehicle or auxiliary into a suitable dosage form.

3. Use of a pharmaceutical composition containing bisoprolol and/or one of its physiologically acceptable salts and a potassium channel activator of the general formula for the preparation of a medicament for lowering blood pressure.

## Claims (Claims for the following Contracting State(s): ES)

1. Process for the preparation of a pharmaceutical composition containing bisoprolol and/or one of its physiologically acceptable salts and a potassium channel activator of the general formula 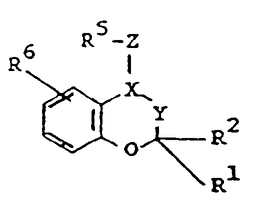 in which
X is -CR⁴-,
Y is -CHR³-,
R¹ and R² are each independently of one another A,
R³ is OH,
R⁴ is H,
R³ and R⁴ together are also a bond,
R⁵ is an oxodihydropyridyl or oxodihydropyridazinyl radical which is unsubstituted or substituted once by A,
R⁶ is CN,
Z is a bond or O, and
A is alkyl with 1-6 C atoms
and/or one of the physiologically acceptable salts thereof, characterized in that bisoprolol and/or one of its physiologically acceptable salts and a potassium channel activator of the general formula are converted together with at least one solid, liquid or semi-liquid vehicle or auxiliary into a suitable dosage form.

## Revendications (Revendications pour l'(les) Etat(s) contractant(s) suivant(s): AT, BE, CH, DE, DK, FR, GB, GR, IT, LI, LU, NL, SE)

1. Préparation pharmaceutique contenant du Bisoprolol et/ou l'un de ses sels physiologiquement acceptables et un activateur de la circulation du potassium de formule générale 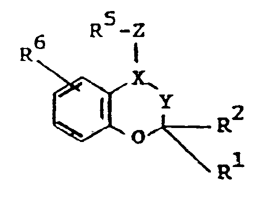 dans laquelle
X représente -CR⁴-,
Y -CHR³-,
R¹ et R² indépendamment l'un de l'autre A
R³ OH
R⁴ H
R³ et R⁴ ensemble également une liaison,
R⁵ un radical oxodihydropyridyle ou oxo-dihydropyridazinyle non substitué ou substitué une fois par A,
R⁶ CN
Z une liaison ou O, et
A un groupe alkyle avec de 1 à 6 atomes de carbone,
et/ou l'un de ses sels physiologiquement acceptables.

2. Procédé de production d'une préparation pharmaceutique, caractérisé en ce que l'on met du Bisoprolol et/ou l'un de ses sels physiologiquement acceptables et un activateur de la circulation du potassium de la formule générale avec au moins un support ou un adjuvant solide, liquide ou semi-liquide sous une forme de dosage appropriée.

3. Utilisation d'une préparation pharmaceutique contenant du Bisoprolol et/ou l'un de ses sels physiologiquement acceptables et un activateur de la circulation du potassium de la formule générale pour préparer un médicament destiné à diminuer la pression sanguine.

## Revendications (Revendications pour l'(les) Etat(s) contractant(s) suivant(s): ES)

1. Procédé de production d'une préparation pharmaceutique contenant du Bisoprolol et/ou l'un de ses sels physiologiquement acceptables et un activateur de la circulation du potassium de formule générale 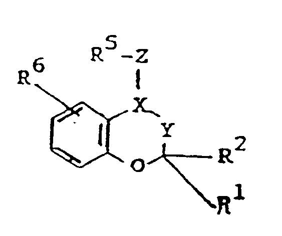 dans laquelle
X représente -CR⁴⁻,
Y -CHR³⁻,
R¹ et R² indépendamment l'un de l'autre A
R³ OH
R⁴ H
R³ et R⁴ ensemble également une liaison,
R⁵ un radical oxodihydropyridyle ou oxo-dihydropyridazinyle non substitué ou substitué une fois par A,
R⁶ CN
Z une liaison ou O, et
A un groupe alkyle avec de 1 à 6 atomes de carbone,
et/ou l'un de ses sels physiologiquement acceptables, caractérisé en ce que l'on met du Bisoprolol et/ou l'un de ses sels physiologiquement acceptables et un activateur de la circulation du potassium de la formule générale avec au moins un support ou un adjuvant solide, liquide ou semi-liquide sous une forme de dosage appropriée.
